# EUROPÄISCHE PATENTSCHRIFT

(11) **EP 4 021 145 B1**
(45) Veröffentlichungstag und Bekanntmachung des Hinweises auf die Patenterteilung: **10.05.2023**
(21) Anmeldenummer: 21190683.9
(22) Anmeldetag: 10.08.2021
(51) Int. Cl.: H05B 6/10, A61B 50/00, A61L 2/00, A61L 11/00

(54) **DOCKINGSTATION FÜR INDUKTIV BEHEIZBARE ABFALLEIMER ZUR INDUKTIVEN DAMPFDEKONTAMINATION**
DOCKING STATION FOR INDUCTIVELY HEATABLE WASTE BUCKET FOR INDUCTIVE STEAM DECONTAMINATION
STATION D'ACCUEIL POUR UN SEAU À ORDURES À CHAUFFAGE INDUCTIF DESTINÉE À LA DÉCONTAMINATION INDUCTIVE À LA VAPEUR

(30) Priorität: 28.12.2020 EP 20217459
(43) Veröffentlichungstag der Anmeldung: 29.06.2022
(73) Patentinhaber: Pink, Tomas, 6215 Beromünster (CH)
(72) Erfinder: Pink, Tomas, 6215 Beromünster (CH)
(74) Vertreter: Raffay & Fleck

(56) Entgegenhaltungen:
- WO-A1-95/24228
- US-A1- 2003 230 567
- US-A1- 2010 151 101
- US-A1- 2011 211 989

## Beschreibung

Die vorliegende Erfindung betrifft eine Vorrichtung und ein Verfahren zur Dampfdekontamination und/oder Dampfsterilisation des Inhalts eines Behälters.

Bekannt sind Abfalleimer mit UV-Lampen aus der CN1 081 00532A.

Auch ist es unter anderem aus der US201 80055595A1, der WO2016109416A1 und der WO2017129848A1 bekannt, Abfallbehälter mit Öffnung in einem Autoklaven zu positionieren und dort durch von außen eindringenden Dampf deren Inhalt zu sterilisieren.

Auch ist aus der CN204433530U und der CN104590793A ein Abfalleimer bekannt, der einen elektrischen Heizer, ein Manometer, einen Wassereinspritzanschluss, einen Wasserstandsmesser und einen Stromversorgungsanschluss aufweist.

Zudem ist ein Autoklav mit Anschlüssen für weitere Autoklavierkammern aus der EP 3 495 001 A1 bekannt.

Auch ist es aus der US 2011 211,989 A1 bekannt, metallisches Material in einem Behälter induktiv zu heizen oder, wie in der US 2003 23,0567 A1 beschrieben, Flüssigkeiten in einem Metallrohr induktiv zu heizen, um sie zu verdampfen und in einen Behälter einzuleiten.

Bestehende Technologien zur effizienten Sterilisation/dekontamination sind teuer, aber auch relativ sperrig und schwer, empfindlich und nicht leicht zu transportieren. Während des Transports können sie beschädigt werden oder beispielsweise die Kalibrierungsparameter der Sensoren beeinträchtigt werden, was einen großen Einfluss auf die Effizienz des Prozesses haben kann. Neben dem Elektroanschluss werden auch andere Energien und Medien gebraucht, z.B. deionisiertes Wasser, Leitungswasser, Druckluft, externe Dampfversorgung. Die Anlagen benötigen auch eine regelmäßige Wartung und den Austausch verschiedener Komponenten nach einer bestimmten Anzahl von Sterilisation/Dekontaminationszyklen.

Andere Lösungen wie die aus der CN108100532A, CN204433530U, CN104590793A bieten zudem nur unzureichende Sterilisation.

Auch ist ein induktiver Milchaufschäumer bekannt, so beispielsweise aus der US 2010/151101 A1.

Die auf dem Markt erhältlichen Geräte können gefährliche Abfälle dekontaminieren, erfüllen zudem jedoch selber nicht die Hygieneanforderungen (EHEDG) und sind beispielsweise nicht für feuchte Umgebungen oder den Einsatz im Freien geeignet. Ihre Konstruktion hat keine IP-Klassifizierung und wenn sie von außen kontaminiert werden, ist es nicht wirklich möglich, sie zu dekontaminieren.

Andere, einfache Methoden, z.B. die chemische Dekontamination/Desinfektion, können nicht die hohe Effizienz bieten und verändern die chemische Zusammensetzung des Abfalls. Was zu weiteren Folgeproblemen führen kann.

Aufgabe der Erfindung ist es, eine Vorrichtung und ein Verfahren zur effizienten, einfachen und zuverlässigen Sterilisation/Dekontamination anzugeben.

Die Erfindung bietet eine technische Lösung, die es möglich macht, verschiedene Arten von biologisch kontaminierten Objekten, Feststoffen, Halbfeststoffen und Flüssigkeiten mit hoher Effizienz sicher und reproduzierbar zu sterilisieren/dekontaminieren, einschließlich Objekte, die bei der Sterilisations- und/oder Dekontaminationstemperatur durch ihren Phasenübergang gehen (schmelzen).

Das System besteht insbesondere aus zwei Teilen. Eines davon ist die Dockingstation, eine Einheit, die für viele Sterilisation/Dekontaminationszyklen immer wieder verwendet werden kann. Diese Einheit ist an die Stromversorgung angeschlossen. Bevorzugt kommuniziert sie mit dem Benutzer (HMI), enthält Steuerelektronik und andere Elemente, die für den Betrieb des Einwegteils, des Behälters, erforderlich sind.

Der Behälter, insbesondere Abfallbehälter, selbst, der zur Sterilisation/Dekontamination in die Dockingstation verbracht wird, wird zur Sammlung, insbesondere Abfallsammlung, verwendet und nach der Sterilisation/Dekontamination entsorgt, insbesondere verbrannt. Der Behälter weist funktionale Elemente auf, die mit der Dockingstation zusammenwirken, um die Sterilisation/Dekontamination zu gewährleisten.

Dabei wird aber nicht von außen Dampf in den Behälter überführt. Vielmehr bleibt der Behälter insbesondere geschlossen und es wird so eine Kontamination der Umgebung effizient verhindert.

Gelöst wird die Aufgabe unter anderem unter Nutzung eines Behälters, insbesondere Abfallbehälters, aufweisend einen zu allen Seiten, insbesondere unter anderem durch Wandung, Boden, Zwischenboden und/oder Deckel, umschlossenen ersten Hohlraum. Dabei ist der erste Hohlraum insbesondere durch eine, insbesondere zu vier Seiten umschließende, Wandung und/oder mindestens einen, insbesondere zu einer fünften Seite verschließenden, Deckel und/oder mindestens einen, insbesondere zu einer sechsten, der fünften gegenüberliegenden Seite umschließenden, Boden und/oder Zwischenboden umschlossen. Der Behälter weist vorteilhafterweise mindestens einen an den ersten Hohlraum angrenzenden, insbesondere unter einem Zwischenboden befindlichen, zweiten Hohlraum auf, innerhalb dessen mindestens ein erstes Heizelement angerordnet ist. Insbesondere sofern kein zweiter Hohlraum vorhanden ist, kann das erste Heizelement auch im ersten Hohlraum angeordnet sein. Insbesondere ist der Zwischenboden ringförmig ausgeführt. Insbesondere ist der mindestens eine zweite Hohlraum ringförmig um einen unteren Abschnitt des ersten Hohlraums angeordnet. Der Behälter ist insbesondere eingerichtet, den ersten Hohlraum luftdicht zu verschließen. Der Behälter ist dadurch gekennzeichnet, dass das mindestens eine erste Heizelement ein induktiv heizbares Heizelement ist. Bevorzugt ist der mindestens eine zweite Hohlraum luftdicht verschlossen und/oder sind Mittel zum Überführen von Dampf aus dem mindesten einen zweiten Hohlraum in den mindestens einen ersten Hohlraum vorgesehen. Die Mittel zur Überführung werden insbesondere dadurch realisiert, dass mindestens ein, insbesondere mindestens vier, insbesondere bei und/oder durch Überdruck und/oder bei und/oder durch Wärme im mindestens einen zweiten Hohlraum gegenüber dem ersten Hohlraum öffnende(n), Durchtritt(e), insbesondere in einem zwischen erstem und mindestens zweitem Hohlraum angeordneten Zwischenboden, aufweist. Das Öffnen erfolgt insbesondere durch Überdruck, insbesondere von mindestens 0,5 barg und/oder maximal 3 barg und/oder durch Temperatur/Wärme, insbesondere bei einer Temperatur im Bereich zwischen 70°C, insbesondere 95°C und 140°C. Dies kann beispielsweise durch Bersten und/oder Schmelzen eines Siegels und/oder Verschlusses erfolgen. Es kann aber auch durch andere Vorkehrungen, beispielsweise ein elektrisch ansteuerbares Ventil erfolgen. Insbesondere beginnt die Erwärmung des mindestens einen zweiten Hohlraums bei geschlossenen Mitteln zum Überführen und/oder bei vollständig geschlossenem mindestens einen zweiten Hohlraum. Insbesondere bleiben die Mittel zum Überführen und/oder der mindestens eine zweite Hohlraum vollständig geschlossen bis im mindestens einen zweiten Hohlraum eine Temperatur von mindestens 40°C und/oder ein Überdruck von mindestens 0,2 barg herrscht und/oder zumindest während des Transports und/oder während des Transports vor Einfüllung von Inhalt, insbesondere Abfall. Der Druck wird dabei immer gegenüber dem ersten Hohlraum bestimmt.

Der zweite Hohlraum kann aber auch geöffnet sein.

In einer alternativen Ausführungsform kann das Wasser in einem dritten Hohlraum, insbesondere des Behälters, umschlossen sein und dieser vor oder durch die Erwärmung geöffnet werden und/oder ausgebildet sein durch die Erwärmung geöffnet zu werden. Insbesondere ist der Behälter so ausgeführt, dass das Wasser aus dem dritten Hohlraum beim Öffnen des dritten Hohlraums auf und/oder um das erste induktiv heizbare Heizelement fließt. Insbesondere kann der dritte Hohlraum durch eine Tüte oder Kapsel gebildet sein, die sich im Behälter befindet.

In einer einfachen Ausführung kann aber auch einfach Wasser in den ersten Hohlraum gefüllt werden, in dem sich das erste induktiv heizbare Heizelement befindet, insbesondere bevor Inhalt, insbesondere Abfall, eingefüllt wird.

Gelöst wird die Aufgabe durch eine Dockingstation zur Aufnahme eines Behälters, insbesondere wie obenstehend beschrieben, wobei die Dockingstation eingerichtet ist, ein elektromagnetisches Feld zum Heizen eines induktiv heizbaren Heizelements zu erzeugen und Mittel zum, insbesondere stützenden, Umschlie-ßen eines Behälters, insbesondere gegen Bersten bei Überdruck im Behälter bis zumindest 3 barg aufweist.

Insbesondere wird die Aufgabe gelöst durch ein System aufweisend mindestens eine erfindungsgemäße Dockingstation und mindestens einen, insbesondere eine Vielzahl, Behälter, insbesondere wie oben beschrieben.

Unter einem induktiv heizbaren Heizelement wird insbesondere ein resistiv heizendes Heizelement verstanden, das eingerichtet ist, in ihm mittels eines elektromagnetischen Feldes einen Strom insbesondere einen Ringstrom, zu induzieren, der dann zu einer resistiven Heizung führt. Das Heizelement ist also insbesondere eingerichtet, elektrischen Strom in Wärme umzuwandeln. Bevorzugt wird mittels eines elektromagnetischen Feldes ein Strom, insbesondere Ringstrom, in dem Heizelement induziert, der genutzt wird, um Wärme zu erzeugen.

Gelöst wird die Aufgabe aber auch unter Nutzung mindestens eines ersten induktiv heizbaren Heizelementes in einem Hohlraum, insbesondere zweiten oder ersten Hohlraum, mit Wasser zur Erzeugung eines Überdrucks in dem umschlossenen Hohlraum und insbesondere zur Öffnung mindestens eines, insbesondere mindestens vier, Durchtritts/en und Einbringen und/oder Erzeugen von Wasserdampf in einen, insbesondere angrenzenden, Aufnahmeraum, insbesondere erster Hohlraum, zur Dampfsterilisierung des Aufnahmeraums und/oder des im Aufnahmeraum befindlichen Inhalts. Das Einbringen erfolgt insbesondere durch den mindestens einen Durchtritt.

Auch ist es für die Dekontamination und/oder Sterilisation, insbesondere im Rahmen einer Entsorgung und/oder zur Abfallentsorgung möglich, mindestens ein erstes induktiv heizbares Heizelement in einem Hohlraum, insbesondere ersten oder zweiten Hohlraum, mit Wasser mittels eines mittels mindestens einer Induktionsspule erzeugten Feld zur Verdampfung des Wassers zu beheizen, so dass ein Überdruck in dem umschlossenen, insbesondere ersten und/oder zweiten, Hohlraum erzeugt wird und, insbesondere durch Öffnung mindestens eines, insbesondere einer Mehrzahl von Durchtritts/en, insbesondere durch die Wärme und/oder den Überdruck im umschlossenen Hohlraum, genutzt und dadurch Wasserdampf in einen/m angrenzenden Aufnahmeraum, insbesondere Abfallaufnahmeraum, insbesondere ersten Hohlraum, zur Dampfsterilisierung des Aufnahmeraums und/oder des im Aufnahmeraum befindlichen Inhalts überführt und/oder erzeugt wird und insbesondere der Aufnahmeraum und der umschlossene Hohlraum anschließend verbrannt werden, wobei die Induktionsspule für die Heizung eines anderen induktiv heizbaren Heizelements, insbesondere eines weiteren erfindungsgemäßen Behälters, verwendet wird.

Mit Vorteil wird in dem zweiten Hohlraum Wasser mittels des ersten induktiv heizbaren Heizelements erhitzt und ein Überdruck erzeugt und Dampf in den Aufnahmeraum überführt. In einer einfachen Ausgestaltung kann das Wasser aber auch im Aufnahmeraum selbst durch ein dort angeordnetes erstes induktiv heizbares Heizelement geheizt und verdampft und direkt im Aufnahmeraum ein Überdruck erzeugt werden.

Bevorzugt wird also mindestens ein erstes induktiv heizbares Heizelement in einem Hohlraum, insbesondere zweiten Hohlraum, mit Wasser zur Erzeugung eines Überdrucks in dem umschlossenen Hohlraum und insbesondere zur Öffnung mindestens eines Durchtritts/en und Einbringen von Wasserdampf in einen angrenzenden Aufnahmeraum, insbesondere erster Hohlraum, zur Dampfsterilisierung des Aufnahmeraums und/oder des im Aufnahmeraum befindlichen Inhalts, verwendet. Der erste Hohlraum kann, insbesondere in einer einfachen Ausführungsform, auch den Aufnahmeraum darstellen.

Insbesondere wird die Verwendung und/oder das Verfahren mit einem oben beschriebenen Behälter, insbesondere Abfalleimer, System und/oder einer erfindungsgemäßen Dockingstation durchgeführt. Insbesondere ist/sind Behälter, Dockingstation und/oder System zur Durchführung des Verfahrens und/oder der Verwendung eingerichtet.

Die umschließende Wandung kann beispielsweise als Zylinderwand aber auch im Querschnitt rund oder viereckig oder mehreckig ausgebildet sein. Sie kann ein- oder mehrteilig ausgeführt sein. Deckel, Boden und/oder Zwischenboden können beispielsweise fest mit der Wandung verbunden und/oder mit dieser einteilig ausgeführt sein. Mit Vorteil sind Wandung und Zwischenboden und/oder der untere Boden gemeinsam einteilig ausgeführt. Dadurch lässt sich die Stabilität erhöhen. Insbesondere ist die Wandung, der Zwischenboden, Boden und/oder Deckel doppelwandig ausgeführt. Das erhöht die Wärmedämmung. Unter Abfall wird insbesondere biologisch kontaminierter Abfall verstanden, z.B. solcher nach Art 3 RL 2008/98/EG. Als induktiv heizbares Heizelement kann beispielsweise ein elektrisch leitender Ring, eine elektrisch leitende Platte oder andere geeignete elektrisch leitende Struktur verwendet werden.

Unter luftdicht verschlossen ist insbesondere zu verstehen, dass der Behälter mit mechanischer Stützung von außen zumindest bis zu einem Innendruck von 1 barg, insbesondere bei 130°C, insbesondere bis zu einem Innendruck von 3 barg, insbesondere bei 130°C, luft- und/oder gasdicht ist.

Die Durchtritte sind insbesondere als Rückschlagventile ausgebildet, die bei einem Überdruck im ersten Hohlraum gegenüber dem mindestens einen zweiten Hohlraum schließen. In einer anderen bevorzugten, besonders einfachen, Ausgestaltung sind sie aus bei der Dampferzeugung im mindestens einen zweiten Hohlraum, insbesondere im Temperaturbereich von 100 bis 120°C, schmelzendem und/oder berstendem Siegel gebildet.

Die Durchtritte sind insbesondere als Düsen ausgebildet, die insbesondere eingerichtet sind, einen gerichteten Dampfstrahl im ersten Hohlraum zu erzeugen. Dadurch lässt sich eine gute Durchmischung im ersten Hohlraum bei der Sterilisation erreichen.

Die Dockingstation kann vorteilhaft sowohl als "Stand Alone Unit" ausgebildet sein oder als "Einbaueinheit" zum Einbau in Schränke, Tisch und/oder Container und/oder in einem Schrank, Tisch und/oder Container eingebaut sein.

Unter "Single-Use"-Material wird in dieser Schrift ein Material oder eine Materialmischung verstanden, die bei einer Entsorgung, insbesondere Deponierung und/oder Verbrennung, insbesondere bei Verbrennung bei Temperaturen im Bereich von 800-1150 °C, insbesondere 800 °C und 900 °C, und/oder bei der Wirbelschichtfeuerung und/oder Rostfeuerung, keine gefährlichen Nebenprodukte oder Emissionen freigeben und entweder Rückstandslos verbrennen und/oder zerfallen oder nur Rückstände hinterlassen, die unbedenklich für Mensch und Umwelt sind und/oder solche, die die Anforderungen der BlmSchG, der BlmSchV und/oder Industrial Emissions Directive 2010/75/EU (Integrated Pollution Prevention and Control) in der zum Anmeldezeitpunkt letzten und/oder gültigen Fassung, insbesondere bei Verbrennung bei Temperaturen im Bereich von 800-1150 °C, insbesondere 800 °C und 900 °C, und/oder bei der Wirbelschichtfeuerung und/oder Rostfeuerung erfüllen. Bevorzugt beinhalten sie keine seltene Elemente oder anders wertvolle Materialien, insbesondere keine Materialien, deren Wert durch die Hitzeeinwirkung um mehr als 50% reduziert wird, insbesondere keine Permanentmagnete. Bevorzugt handelt es sich um Kunststoff, Emaille, Keramik, Stahl, Wolframkarbid, Titannitrid, Aluminiumoxid, Metall- und Halbmetall-Nitride, Metall-Carbide, Metall- und Halbmetall-Oxide, Glas- und/oder Kohlenstoffverbundwerkstoff, Glas- und/oder Kohlenstofffasern, Aramid, Aramidharz und/oder Aluminium und Aluminiumlegierungen, Magnesiumlegierungen, bevorzugt um Kunststoff. Als Kunststoff kommt beispielsweise in Betracht Polypropylen, Polyethylen, Polyvinylacetat, Polyethylvinylacetat, PEEK, PET, Polyamide und Polyimide.

Mit besonderem Vorteil beinhaltet der Behälter mindestens einen Sensor, insbesondere mindestens einen Temperatur und/oder Drucksensor, wobei der mindestens eine Sensor zur drahtlosen Übertragung von Messwerten und/oder zur drahtlosen Stromspeisung, insbesondere zur Messung und/oder Übertragung von Messwerten, eingerichtet ist und/oder der mindestens eine Sensor und/oder seine Mittel zum Übertragen von Messwerten und/oder zur drahtlosen Stromspeisung in der oberen Hälfte des Behälters angeordnet sind. Mit derartigen Sensoren lässt sich der Prozess überwachen und/oder darauf basierend steuern und/oder regeln. Insbesondere sind die Übertragungsmittel zum Übertragen der Messwerte, beispielsweise mindestens eine Antenne, von den induktiv heizbaren Heizelementen entfernt, mindestens 10 cm entfernt, insbesondere in der oberen Hälfte, insbesondere in dem oberen Fünftel, des Behälters angeordnet. Oben ist insbesondere das von dem mindestens einen ersten induktiv heizbaren Heizelement entfernte und/oder dem gegenüberliegende Ende des Behälters. Dadurch lassen sich Störungen durch die Induktion vermeiden. Der mindestens eine Sensor ist mit den Mitteln zur Übertragung insbesondere mittels mindestens einem Kabel und/oder elektrischen Leiter verbunden. Mit besonderem Vorteil ist mindestens ein Druck- und/oder Temperatursensor zur Messung des Drucks und/oder der Temperatur im obersten Drittel, insbesondere im obersten Fünftel, des ersten Hohlraums vorgesehen. Bevorzugt wird mindestens ein Temperatursensor zur Messung der Temperatur im untersten Drittel, insbesondere im untersten Fünftel, des ersten Hohlraums vorgesehen. So lässt sich der Prozess besonders zuverlässig überwachen und/oder steuern und/oder regeln. Mit besonderem Vorteil ist ein Flüssigkeitsniveausensor zur Bestimmung des Flüssigkeitsniveaus im ersten Hohlraum im Behälter und/oder Dockingstation angeordnet.

Mit Vorteil ist der mindestens eine zweite Hohlraum zumindest teilweise mit Wasser gefüllt. Er ist insbesondere vor Befüllung des Behälters mit Inhalt, insbesondere Abfall, mit Wasser gefüllt. Das Wasser ist insbesondere vollständig im mindestens einen zweiten Hohlraum eingeschlossen. Insbesondere weist der mindestens eine zweite Hohlraum keine Öffnungen auf. Insbesondere weist er neben den Mitteln zum Überführen keine Öffnungsmittel auf. Insbesondere sind pro 1 I Volumen des ersten Hohlraums mindestens 0,80 ml, insbesondere mindestens 2 ml, Wasser in dem mindestens einen zweiten Hohlraum vorhanden. Insbesondere sind pro 1 I Volumen des ersten Hohlraums maximal 5 ml, insbesondere maximal 100 ml, Wasser in dem mindestens einen zweiten Hohlraum vorhanden. Sind mehrere zweite Hohlräume vorhanden, sind für diese Betrachtung die Wassermengen in allen zweiten Hohlräumen zu addieren. Mit diesen Wassermengen lässt sich besonders effizient eine Sterilisation/Dekontamination erreichen.

Mit besonderem Vorteil ist der Behälter und/oder die Wandung, der Boden, Zwischenboden und/oder der Deckel aus "Single-Use"-Material, insbesondere Kunststoff, insbesondere Polypropylen und/oder "Glass Fibre filled" Polypropylen, gebildet. Dies ist besonders Ressourcen schonend und einfach in der Herstellung.

Bevorzugt ist der Behälter so ausgebildet, dass er ohne Unterstützung von außen einem Innendruck von 3 barg bei 130°C nicht widersteht. Dies spart Konstruktionsmaterial des Behälters, was widerum besonders Ressourcen schonend ist und der Behälter ist dadurch leicht in Handhabung und Transport. Die Widerstandskraft gegen 3 barg und mehr kann durch Unterstützung, insbesondere durch die Dockingstation, erreicht werden.

Bevorzugt ist der Abfallbehälter so ausgebildet, dass er bei mechanischer Stützung und/oder Umschließung, insbesondere durch die Umschließungsmittel der Dockingstation mindestens 3 barg bei 130°C Innendruck im ersten und/oder zweiten Hohlraum widersteht. So kann eine zuverlässige Dampfsterilisation/-dekontamination ermöglicht werden.

Mit besonderem Vorteil weist der Behälter einen Druckübertragungsbereich, insbesondere eine Membran, insbesondere in der Wandung auf, der so ausgebildet ist, dass er es ermöglicht, den Innendruck im ersten und/oder zweiten Hohlraum von außen zu messen. Dazu weist die Dockingstation insbesondere einen Drucksensor zum Andrücken an diesen Druckübertragungsbereich während des Prozesses auf, um den Druck im ersten Hohlraum zu messen. Insbesondere ist sie zum Andrücken des Drucksensors, insbesondere an einen Druckübertragungsbereich ausgebildet. Dadurch lässt sich der Drucksensor mehrfach mit verschiedenen Behältern verwenden.

Bevorzugt weist der Behälter mindestens ein zweites induktiv heizbares Heizelement auf. Insbesondere ist er so ausgebildet, dass an den Wandungen des Behälters im Inneren des Behälters herabfließendes Kondensat aufgefangen und so zum mindestens einen ersten oder zweiten Heizelement geleitet wird, dass es mit dem mindestens einem ersten oder zweiten Heizelement erwärmt werden kann, insbesondere mit diesem in Kontakt gebracht wird. Dadurch lässt sich die Verdampfung von Kondensat und/oder Flüssigkeit im ersten Hohlraum zuverlässig erreichen und die Wirksamkeit der Dampfsterilisation/-dekontamination steigern.

Das erste und/oder zweite Heizelement ist/sind bevorzugt aus "Single-Use"-Material, aus Metall und/oder Metalllegierung, insbesondere ist/sind es/sie aus Aluminium oder Aluminiumlegierung gebildet und/oder ist der Behälter ausschließlich aus "Single-Use"-Material gebildet.

Mit besonderem Vorteil weist der Behälter einen verschließbaren Einwurf, insbesondere im Deckel und/oder in der Wandung auf. So ist die Befüllung einfach möglich, eine Kontaminierung der Umgebung durch Verschließen des Einwurfs aber vermeidbar und/oder reduzierbar.

Bevorzugt ist der Behälter als Abfalleimer ausgeführt. So ist die Befüllung einfach möglich.

Mit Vorteil weist der Behälter und/oder der erste Hohlraum ein Volumen im Bereich von 200 ml oder mehr und/oder von 200 I oder weniger auf. Diese Größen haben sich als besonders praktisch und geeignet herausgestellt.

Mit großem Vorteil ist der Behälter eingerichtet, so verschlossen zu werden, dass keine Öffnungen nach außen und/oder keine Durchführungen nach außen mit Ausnahme einer Abgasauslassöffnung aus dem ersten Hohlraum aus dem Behälter hinaus vorhanden sind.

Mit Vorteil weist der Behälter eine Abgasauslassöffnung vom ersten Hohlraum nach außen auf, in der sich insbesondere ein Auslassventil und/oder ein Sterilfilter, beispielweise ein Membranfilter oder ein Tiefenfilter, befindet. Durch eine solche Abgasauslassöffnung lassen sich, insbesondere nach der Dampfsterilisation/-dekontamination, gezielt und ungefährlich Überdrücke abbauen und/oder während der Dampfsterilisation/-dekontamination Abgase abführen. Durch ein Ventil lässt sich dies gezielt steuern, durch einen Filter lässt sich, vor allem zu Beginn der Dampfsterilisation/-dekontamination, das Entweichen von Kontamination verhindern.

Mit besonderem Vorteil ist die Abgasauslassöffnung mit einem flexiblen, insbesondere TPE, Schlauch mit dem ersten Hohlraum verbunden und/oder das Auslassventil als Schlauchquetschventil ausgeführt. So lässt sich besonders einfach und Ressourcen schonend ein Ventil realisieren. Auch kann die Abgasauslassöffnung mit einem Ventil, beispielsweise Membranventil, verbunden sein, wobei das Ventil insbesondere von außen, insbesondere durch Mittel der Dockingstation, mechanisch ansteuerbar und/oder mechanisch bedienbar eingerichtet ist. Insbesondere weist die Dockingstation entsprechende Mittel auf.

Bevorzugt weist der Behälter keine Kabeldurchführungen und/oder keine Stromanschlüsse, insbesondere nach außen, und/oder keine elektrischen Kontakte, insbesondere außen, auf. Dadurch lässt sich die Sicherheit erhöhen.

Mit Vorteil weist der Behälter abgesehen von einer Abgasauslassöffnung keine Verbindung von erstem und/oder zweitem Hohlraum nach außen auf, insbesondere wenn der verschließbare Einwurf geschlossen ist und/oder während der Dampfsterilisation/-dekontamination.

Mit besonderem Vorteil weist die Dockingstation Mittel zur Erzeugung eines Unterdrucks in dem Behälter, in seinem ersten Hohlraum, auf, z. B. eine Vakuumpumpe, ein Gebläse, eine Membranpumpe, eine ScrollPumpe, eine Liquid Ring Pumpe, eine Klauenpumpe und/oder ist er ausgebildet zumindest einem Unterdruck von 0,5 barg Stand zu halten. Dadurch lassen sich Flüssigkeiten in den ersten Hohlraum saugen und/oder das Entweichen von Partikeln reduzieren.

Mit besonderem Vorteil weist der Behälter Mittel zum Anschluss von Mitteln zur Erzeugung eines Unterdrucks im ersten Hohlraum auf, z. B. eine Durchführung und/oder einen Schlauch zum Anschluss dieser Mittel. Mit besonderem Vorteil weisen die Mittel zum Anschluss einen, insbesondere hydrophoben, Sterilfilter, beispielweise einen Membranfilter oder einen Tiefenfilter auf, wobei die Mittel zum Anschluss so ausgebildet sind, dass aus dem Behälter durch die Mittel zum Anschluss abgesaugte Luft durch den Sterilfilter geleitet werden.

Mit besonderem Vorteil ist der mindestens eine zweite Hohlraum in mehrere Hohlräume unterteilt und/oder sind mehrere zweite Hohlräume vorgesehen. Insbesondere ist pro zweitem Hohlraum und/oder pro Unterteilung mindestens ein, insbesondere genau ein, Durchtritt und/oder mindestens ein zweites induktiv heizbares Heizmittel vorgesehen. Die Durchtritte sind insbesondere zwischen dem ersten Hohlraum und jedem zweiten Hohlraum und/oder jeder Unterteilung vorgesehen. Dadurch kann sichergestellt werden, dass alle Durchtritte, insbesondere wenn sie durch Überdruck im zweiten Hohlraum öffnen, öffnen. Die Unterteilung und/oder das mindestens eine zweite induktiv heizbare Heizmittel sind insbesondere so ausgebildet, dass in jeder Unterteilung ein ausreichender Druck zum Öffnen des jeweiligen Durchtritt durch Erhitzung mittels des induktiv heizbaren zweiten Heizelements aufgebaut werden kann.

Die Aufnahme in der Dockingstation kann beispielsweise durch Verrasten und/oder zumindest teilweises Umschließen erfolgen. Die Dockingstation kann zum Erzeugen des elektrischen, insbesondere elektromagnetischen, Felds zum Heizen eines induktiv heizbaren Heizelements zum Beispiel mindestens eine Induktionsspule aufweisen. Die Mittel zum, insbesondere stützenden, Umschließen eines Behälters, insbesondere gegen Bersten bei Überdruck im Behälter bis zumindest 3 barg und/oder als Sicherheitsbarriere können beispielweise durch Elemente zum formschlüssigen zumindest teilweisen Umschließen des Behälters, beispielsweise aus Metall, gebildet sein. Die können aber auch so ausgebildet sein, den Behälter nur an einer Vielzahl kleiner Bereiche mechanisch zu stützen.

Die Mittel zum Umschließen können aber auch allein als Schutz bei zerberstendem Behälter und/oder als thermische Isolation ausgebildet sein und/oder solche Elemente aufweisen. Besonders bevorzugt wird es, wenn der Behälter durch die Dockingstation vollständig umschlossen werden kann und/oder diese dazu ausgebildet ist.

Mit besonderem Vorteil sind die Mittel zum Umschließen und/oder die Dockingstation zusammenklappbar, -schiebbar und/oder -faltbar ausgebildet, sodass die Ausmaße der Dockingstation durch Zusammenklappen, - schieben und/oder -falten der Mittel zum stützenden Umschließen und/oder der Dockingstation verringert werden können, insbesondere so, dass die Ausmaße kleiner sind als der maximal aufnehmbare Behälter.

Mit Vorteil weist die Dockingstation mindestens einen Sensor, insbesondere Druck- und/oder Temperatursensor, auf, der so angeordnet ist, dass bei Aufnahme eines Behälters und Umschließen des Behälters, insbesondere mit den Mitteln zum Umschließen, der mindestens eine Sensor an den Behälter gedrückt wird. Als Sensor kann beispielsweise ein JUMO dTRANS p31 genutzt werden, dessen Edelstahlmembran insbesondere an eine flexible Plastik-Membran des Behälters angelegt und/oder angedrückt wird.

Als Sensor aus "Single-Use" Material kann beispielsweise ein PendoTECH "Single Use Pressure Sensor" verwendet werden.

Bevorzugt weist die Dockingstation Mittel zum Empfangen von drahtlos übertragenen Messdaten, insbesondere des Behälters, auf und/oder weist sie Mittel zur drahtlosen Stromspeisung von Sensoren und/oder Kommunikationsmitteln, insbesondere im Behälter, auf.

Mit Vorteil weist die Dockingstation Mittel zum Versiegeln und/oder Markieren eines aufgenommenen und/oder sterilisierten/dekontaminierten Behälters auf. So lässt sich der Prozess besonders sicher gestalten.

Bevorzugt weist die Dockingstation Mittel zum Erkennen und/oder Auslesen einer Identifikation eines Behälters und/oder Speichern einer solchen auf. Dadurch lässt sich der Prozess, insbesondere auch durch Dokumentation, sicherer gestalten.

Mit besonderem Vorteil weist die Dockingstation mindestens einen Anschluss zum Anschluss und/oder eine Aufnahme zur Aufnahme eines Abgasauslasses eines aufgenommenen Behälters auf. Der Anschluss ist insbesondere mit einem Sterilfilter, beispielweise einem Membranfilter oder einem Tiefenfilter strömungstechnisch so verbunden, dass in den Anschluss eingeführtes Luft/Kondensat/Dampfgemisch durch den Sterilfilter geleitet wird. Dadurch lässt sich die Dampfsterilisation/-dekontamination besonders sicher gestalten. Verwendete Filter/Membranen, insbesondere hydrophobe, können zunächst Kontaminationen in Abgasen zurückhalten und zum Ende der Dampfsterilisation/-dekontamination selbst durch das durch sie geleitete Luft/Dampf/Kondensatgemisch dekontaminiert werden, da das Luft/Dampf/Kondensatgemisch in sie eindringen kann. Insbesondere sind die Filter/Membranen entsprechend ausgebildet. Diese hydrophob auszubilden erleichtert die Sterilisation/Dekontamination, da Kondensat von ihnen abtropft oder abläuft.

Bevorzugt weist die Dockingstation, insbesondere die Aufnahme, Mittel zum Öffnen und/oder Schließen eines Abgasventils des Behälters, insbesondere des Abgasauslasses des Behälters auf, insbesondere Mittel zum Quetschen und/oder Öffnen und/oder Schließen eines Ventils eines aufgenommenen Behälters, insbesondere eines flexiblen, insbesondere TPE, Schlauchs und/oder Schlauchquetschventils des Behälters. In einer anderen Ausgestaltung kann die Aufnahme und/oder die Dockingstation ein Ventil aufweisen, wobei der Anschluss mit dem Ventil strömungstechnisch so verbunden ist, dass in den Anschluss eingeführtes Luft-/Dampf- und/oder Kondensatgemisch auf das Ventil geleitet wird. Dadurch lässt sich die Dampfsterilisation/-dekontamination besonders sicher gestalten und besonders zuverlässig durchführen.

Mit Vorteil ist die Dockingstation eingerichtet, oberflächendekontaminiert zu werden. Dazu weist sie insbesondere eine Ingress Protection (IP) mindestens auf dem Level 68 auf. Insbesondere weist sie keine Spalten, keine Poren und/oder keine Risse auf, in denen sich Kontamination ansammeln können. Bevorzugt weist sie keine offenen Elektrokontakte und/oder keine korrodierenden Teile auf.

Mit besonderem Vorteil erfolgt die Dampfsterilisation/-dekontamination und/oder Heizung ausschließlich durch die induktiv heizbaren Heizelemente des Behälters. Insbesondere ist die Dockingstation dazu eingerichtet, die Dampfsterilisation/-dekontamination des ersten Hohlraums des aufgenommenen Behälters ausschließlich mittels induktiv heizbarer Heizelemente des Behälters durchzuführen. Insbesondere weist die Dockingstation keine Heizmittel, Heizer und/oder Dampferzeuger auf. Insbesondere weist der Behälter keine anderen Heizmittel und/oder Dampferzeuger auf als die ersten und/oder zweiten induktiv heizbaren Heizmittel.

Eine mögliche Ausführungsform der Erfindung soll im Folgenden rein schematisch und nicht beschränkend anhand der folgenden Figur erläutert werden.

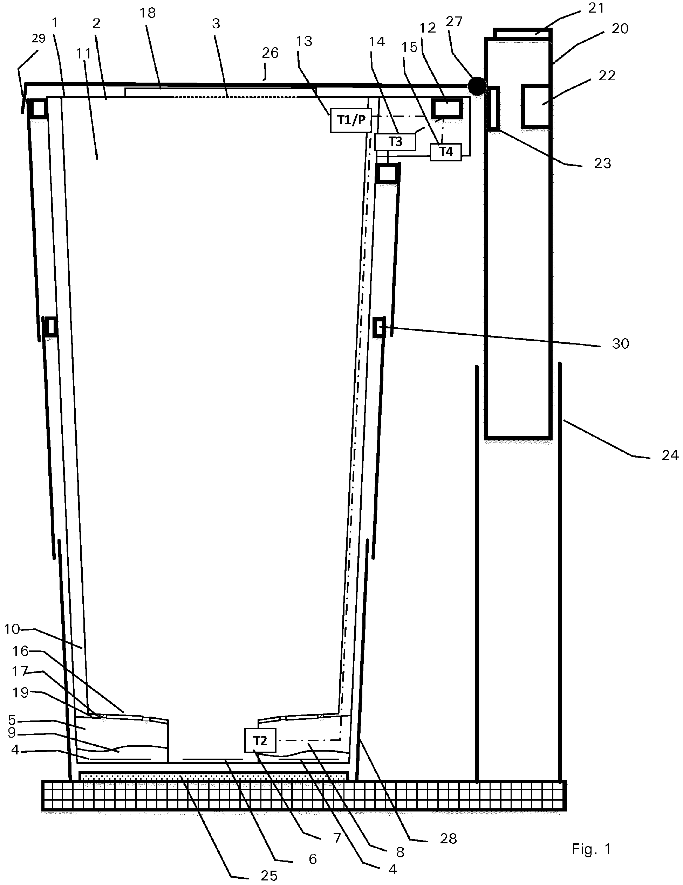

Figur 1 zeigt einen Schnitt durch eine Dockingstation 20, in der ein Behälter 1 aufgenommen ist. Zu erkennen ist ein Behälter 1, der eine doppelwandige Wandung aufweist. Die innere Wand 10 grenzt einen ersten Hohlraum 11 zu vier Seiten ab.

Nach unten hin ist er an den Seiten durch einen Zwischenboden 19 begrenzt. In der Mitte ist der Zwischenboden 19 mit dem Boden des Behälters identisch. Der Behälter weist radial angeordnet mehrere zweite Hohlräume 5 auf. Diese zweiten Hohlräume 5 sind komplett geschlossen. In ihnen ist jeweils ein erstes Heizelement 4 angeordnet, dass induktiv beheizt werden kann. Zudem ist in jedem zweiten Hohlraum 5 Wasser 9 angeordnet. Im Zwischenboden 19 sind für jeden zweiten Hohlraum 5 Ventile 17 mit Düsen in Richtung des ersten Hohlraums 11 angeordnet. Darüber hinaus verfügt der erste Hohlraum 11 an seiner tiefsten Stelle über ein induktiv heizbares zweites Heizelement 6. Zudem ist der Zwischenboden 19 so ausgebildet, dass er einen Ablauf 16 für Kondenswasser in Richtung des zweiten induktiv heizbaren Heizelement 6 darstellt.

Der Behälter 1 weist einen Einwurf 3 auf, der mittels eines Verschlusses 18 verschlossen ist.

Zudem weist der Behälter 1 mehrere Sensoren 13, 14, 15 sowie eine drahtlose Kommunikationseinrichtung 12 auf. Die drahtlose Kommunikationseinrichtung ist mit den Sensoren über elektrisch leitende Kabel verbunden. Die Sensoren beinhalten einen Temperatur und einen Drucksensor 13 zum Messen der Temperatur und des Drucks im oberen Bereich des ersten Hohlraums 11, einen Temperatursensor 15 zur Messung der Temperatur im Innenbereich der Wandung des Behälters 1, einen Temperatursensor 14 zum Messen der Temperatur der Abluftstrecke/am Sterilfilter sowie einen Temperatursensor 7 zur Messung der Temperatur im unteren Bereich des ersten Hohlraums 11. Die Dockingstation 20 weist ein Bedieninterface 21 zur Interaktion mit dem Benutzer sowie eine Kommunikationseinrichtung 22 zu Kommunikation mit anderen Bedienelementen, wie Tabletts und/oder Netzwerk auf. Die Kommunikationseinrichtung 22 ist insbesondere zur drahtlosen Kommunikation ausgestaltet.

Die Dockingstation 20 ist höhenverstellbar ausgestaltet und weist dazu eine Höhenverstellung 24 auf, mit der die Höhe der Dockingstation 20 verändert werden kann, dies insbesondere zum Verkleinern der Dockingstation zum Transport und zur Lagerung, sie kann aber auch zur Anpassung an unterschiedlich hohe Behälter 1 genutzt werden. Zudem verfügt die Dockingstation über mindestens eine Induktionsspule 25 zur induktiven Heizung der Heizelemente 4, 6. Darüber hinaus verfügt die Dockingstation über eine drahtlose Kommunikationseinrichtung 22 zur Kommunikation mit der drahtlosen Kommunikationseinrichtung 12 des Behälters 1. Zudem verfügt sie über eine als Isolationswand und Unterstützung ausgebildete Abdeckung 26, die über ein Scharnier 27 schwenkbar ist und an ihrem dem Scharnier 27 gegenüberliegenden Ende eine Verriegelung mit der höhenverstellbaren Isolationswand 28 aufweist. Die Isolationswand 28 weist zudem Unterstützungsfortsätze 30 auf, um einen aufgenommenen Behälter 1 mechanisch zu stützen und dadurch druckresistenter zu machen.

Die Dockingstation 20 ist eingerichtet, mittels der Induktionsspule 25 die induktiv heizbaren Heizelemente 4, 6 des Behälters 1 zu heizen und dadurch das Wasser 9 zum Verdampfen zu bringen und die Ventile 17 zu öffnen und Dampf in den ersten Hohlraum 11 zu überführen. Dabei werden Druck und Temperatur durch die Sensoren 13, 14, 15 gemessen und die Messwerte über die drahtlose Kommunikationseinrichtung 12 zur drahtlosen Kommunikationseinrichtung 23 übertragen. Zudem ist die Dockingstation 20 eingerichtet, mittels der drahtlosen Kommunikationseinrichtung 23 Energie zur drahtlosen Kommunikationseinrichtung 12 des Behälters 1 zu übertragen und damit die Energie für die Messungen und die Übertragung der Messwerte zu übertragen. Kondensat, dass sich zum Beispiel an der Wandung 10 des ersten Hohlraums 11 sammelt kann über den schrägen Ablauf 16 auf die zweite Induktionsheizplatte 6 geleitet und dort wieder verdampft werden.

### Bezugszeichenliste

- 1: Behälter
- 2: Deckel
- 3: Einwurföffnung
- 4: erstes induktiv heizbares Heizelement
- 5: zweiter Hohlraum
- 6: zweites induktiv heizbares Heizelement
- 7: Temperatursensor
- 8: elektrische Leitung
- 9: Wasser
- 10: innere Wand
- 11: erster Hohlraum
- 12: drahtlose Kommunikationseinrichtung
- 13: Temperatur und Drucksensor
- 14: Temperatursensor
- 15: Temperatursensor
- 16: Ablauf
- 17: Ventil
- 18: Verschluss
- 19: Zwischenboden
- 20: Dockingstation
- 21: Bedieninterface
- 22: Kommunikationseinrichtung
- 23: drahtlose Kommunikationseinrichtung
- 24: Höhenverstellung
- 25: Induktionsspulen
- 26: Abdeckung
- 27: Scharnier
- 28: Isolationswand
- 29: Verriegelung
- 30: Unterstützungsfortsatz

## Patentansprüche

1. Dockingstation (20) zur Aufnahme eines Behälters (1), insbesondere Abfallbehälters, aufweisend einen zu allen Seiten, umschlossenen ersten Hohlraum (11), wobei der Behälter (1) ein erstes induktiv heizbares Heizelement (4) aufweist, wobei die Dockingstation eingerichtet ist, ein elektromagnetisches Feld zum Heizen eines induktiv heizbaren Heizelements zu erzeugen **dadurch gekennzeichnet, dass** die Dockingstation Mittel (28, 30) gegen Bersten bei Überdruck im aufgenommenen Behälter bis zumindest 3 barg aufweist.

2. Dockingstation nach dem vorstehenden Anspruch 1, wobei die Mittel zum Umschließen Mittel zur thermischen Isolation eines aufgenommenen Behälters aufweisen.

3. Dockingstation nach einem der vorstehenden Ansprüche 1 bis 2, wobei die Dockingstation mindestens einen Sensor, insbesondere Druck- und/oder Temperatursensor, aufweist, der so angeordnet ist, dass er bei Aufnahme eines Behälters und umschließen des Behälters mit den Mitteln zum stützenden Umschließen der mindestens eine Sensor an den Behälter gedrückt wird und/oder wobei die Dockingstation Mittel (23) zum Empfangen von drahtlos übertragenen Messdaten, insbesondere des Behälter, aufweist und/oder zur drahtlosen Stromspeisung von Sensoren, insbesondere im Behälter, eingerichtet ist.

4. Dockingstation nach einem der vorstehenden Ansprüche1 bis 3, wobei die Dockingstation mindestens einen Anschluss zum Anschluss und/oder eine Aufnahme zur Aufnahme eines Abgasauslasses eines aufgenommenen Behälters aufweist, und wobei dieser Anschluss insbesondere mit einem Sterilfilter strömungstechnisch so verbunden ist, dass in den Anschluss eingeführtes Luft/Dampf/Kondensatgemisch durch den Sterilfilter geleitet wird und/oder die Aufnahme zum Öffnen und/oder Schließen eines Abgasventils des Behälters, insbesondere des Abgasauslasses des Behälters, insbesondere zum Quetschen und/oder Öffnen eines flexiblen, insbesondere TPE, Schlauchs und/oder Schlauchquetschventils des Behälters ausgebildet ist und/oder ein Ventil aufweist, wobei der Anschluss mit dem Ventil strömungstechnisch so verbunden ist, dass in den Anschluss eingeführtes Luft/Dampf/Kondensatgemisch auf und/oder durch das Ventil geleitet wird.

5. System aufweisend mindestens eine Dockingstation (20) nach einem der vorstehenden Ansprüche 1 bis 4 und mindestens einen Behälter (1), insbesondere Abfallbehälter, aufweisend einen zu allen Seiten, umschlossenen ersten Hohlraum (11), wobei der Behälter (1) ein erstes induktiv heizbares Heizelement (4) aufweist.

6. Verfahren zur Dekontamination und/oder Sterilisation, wobei ein Behälter (1), insbesondere Abfallbehälter, aufweisend einen zu allen Seiten, umschlossenen ersten Hohlraum (11) sowie ein erstes induktiv heizbares Heizelement (4), zur Sammlung, insbesondere Abfallsammlung, verwendet wird und anschließend zur Sterilisation und/oder Dekontamination in eine Dockingstation nach einem der Ansprüche 1 bis 4 verbracht wird, und nach der Sterilisation und/oder Dekontamination entsorgt, insbesondere verbrannt, wird.

7. Verfahren nach Anspruch 6, wobei das Sammeln des Inhalts in dem Behälter von der Dockingstation getrennt erfolgt.

8. Verfahren nach einem der vorstehenden Ansprüche 6 bis 7, wobei die Sterilisation und/oder Dekontamination mittels Heizen des induktiv heizbaren Heizelements durch das durch die Dockingstation erzeugte elektromagnetische Feld erfolgt, insbesondere mittels mit dem induktiven Heizelement erzeugten Wasserdampf.

9. Verfahren nach einem der vorstehenden Ansprüche 6 bis 8, wobei die Sterilisation und/oder Dekontamination eine Sterilisation und/oder Dekontamination des ersten Hohlraums und/oder des im ersten Hohlraum aufgenommenen Materials, insbesondere Abfalls, darstellt.

10. Verfahren nach einem der vorstehenden Ansprüche 6 bis 9, wobei nach der Sterilisation und/oder Dekontamination der Behälter von der Dockingstation getrennt und/oder entfernt wird und der Behälter entsorgt wird und/oder die Dockingstation nicht entsorgt wird.

11. Verfahren nach einem der vorstehenden Ansprüche 6 bis 10, wobei die Dockingstation, insbesondere nacheinander, zur Sterilisation und/oder Dekontamination mehrerer erster Hohlräume je eines Behälters und/oder des darin aufgenommenen Materials, insbesondere Abfalls, verwendet wird, wobei die Behälter je aufweisen einen zu allen Seiten, umschlossenen ersten Hohlraum (11) sowie ein erstes induktiv heizbares Heizelement (4), wobei die Behälter nach der Sterilisation und/oder Dekontamination entsorgt, insbesondere verbrannt, werden, insbesondere nachdem sie jeweils von der Dockingstation entfernt und/oder getrennt wurden.

12. Verfahren nach einem der vorstehenden Ansprüche 6 bis 11, wobei während der Sterilisation und/oder Dekontamination drahtlos von mindestens einem Sensor des Behälters mindestens eine, insbesondere mehrere, Druck- und/oder Temperaturmesswert(e) erhalten, insbesondere angefragt, wird/werden und insbesondere das elektromagnetische Feld, insbesondere dessen Stärke und/oder Dauer, insbesondere durch die Dockingstation, zumindest auch abhängig von dem mindestens einen erhaltene Druck- und/oder Temperaturmesswert gesteuert, insbesondere geregelt, wird.

13. Verfahren nach einem der vorstehenden Ansprüche 6 bis 12, wobei die Sammlung durch Eingeben und/oder Einsaugen von festem und/oder flüssigem Material, insbesondere Abfall, in den ersten Hohlraum erfolgt.

## Claims

1. A docking station (20) for receiving a container (1), in particular a waste container, having a cavity (11) enclosed on all sides, the container (1) having a first inductively heatable heating element (4), the docking station being arranged to generate an electromagnetic field for heating an inductively heatable heating element, **characterised in that** the docking station has means (28, 30) for preventing bursting in the event of overpressure in the received container up to at least 3 barg.

2. A docking station according to claim 1, wherein said means for enclosing comprises means for thermally insulating a received container.

3. A docking station according to any one of the preceding claims 1 to 2, wherein the docking station has at least one sensor, in particular a pressure and/or temperature sensor, which is arranged in such a way that, when a container is received and the container is enclosed by the means for supporting enclosure, said at least one sensor is pressed against the container and/or wherein the docking station has means (23) for receiving wirelessly transmitted measurement data, in particular of the container, and/or is set up for wireless power supply to sensors, in particular in the container.

4. A docking station according to one of the preceding claims to 3, wherein the docking station has at least one connection for connecting and/or a receptacle for receiving an exhaust gas outlet of a received container, and wherein this connection is fluidically connected in particular to a sterile filter in such a way that air/steam/condensate mixture introduced into the connection is conducted through the sterile filter and/or the receptacle for opening and/or closing an exhaust gas valve of the container, in particular the exhaust gas outlet of the container, in particular for squeezing and/or opening a flexible, in particular TPE, hose and/or hose squeezing valve of the container and/or has a valve, the connection being fluidically connected to the valve in such a way that air/ vapour/condensate mixture introduced into the connection is passed onto and/ or through the valve.

5. A system comprising at least one docking station (20) according to one of the preceding claims 1 to 4 and at least one container (1), in particular waste container, comprising a first cavity (11) enclosed on all sides, wherein the container (1) comprises a first inductively heatable heating element (4).

6. A method for decontamination and/or sterilisation, wherein a container (1), in particular a waste container, having a first cavity (11) enclosed on all sides and a first inductively heatable heating element (4), is used for collection, in particular waste collection, and is then brought into a docking station according to one of claims 1 to 4 for sterilisation and/or decontamination, and is disposed of, in particular incinerated, after the sterilisation and/or decontamination.

7. A method according to claim 6, wherein the collection of the contents in the container is performed separately from the docking station.

8. A method according to any one of the preceding claims 6 to 7, wherein the sterilisation and/or decontamination is performed by means of heating the inductively heatable heating element by the electromagnetic field generated by the docking station, in particular by means of steam generated with the inductive heating element.

9. A method according to any one of the preceding claims 6 to 8, wherein the sterilisation and/or decontamination constitutes a sterilisation and/or decontamination of the first cavity and/or of the material, in particular waste, received in the first cavity.

10. A method according to any one of the preceding claims 6 to 9, wherein after the sterilisation and/or decontamination the container is separated and/or removed from the docking station and the container is disposed of and/or the docking station is not disposed of.

11. A method according to any one of the preceding claims 6 to 10, wherein the docking station is used, in particular successively, for sterilising and/or decontaminating a plurality of first cavities of a respective container and/or the material, in particular waste, accommodated therein, wherein the containers each comprise a first cavity (11) enclosed on all sides and a first inductively heatable heating element (4), wherein the containers are disposed of, in particular incinerated, after the sterilisation and/or decontamination, in particular after they have each been removed and/or separated from the docking station.

12. A method according to one of the preceding claims 6 to 11, wherein during the sterilisation and/or decontamination at least one, in particular several, pressure and/or temperature measured value(s) is/are received, in particular requested, wirelessly from at least one sensor of the container and in particular the electromagnetic field, in particular its strength and/or duration, is controlled, in particular regulated, in particular by the docking station, at least also depending on said at least one pressure and/or temperature measured value received.

13. A method according to any one of the preceding claims 6 to 12, wherein the collection is performed by feeding and/or sucking solid and/or liquid material, in particular waste, into the first cavity.

## Revendications

1. Station d'accueil (20) destinée à recevoir un conteneur (1), notamment un conteneur à déchets, présentant une cavité (11) fermée de tous côtés, le conteneur (1) présentant un premier élément chauffant inductif (4), la station d'accueil étant agencée pour générer un champ électromagnétique destiné à chauffer un élément chauffant inductif, **caractérisée en ce que** la station d'accueil présente des moyens (28, 30) pour empêcher l'éclatement en cas de surpression dans le conteneur reçu, jusqu'à au moins 3 barg.

2. Station d'accueil selon la revendication 1, dans laquelle lesdits moyens d'enfermement comprennent des moyens d'isolation thermique d'un conteneur reçu.

3. Station d'accueil selon l'une quelconque des revendications précédentes 1 à 2, dans laquelle la station d'accueil comporte au moins un capteur, en particulier un capteur de pression et/ou de température, qui est disposé de telle sorte que, lorsqu'un conteneur est reçu et que le conteneur est enfermé par les moyens de support de l'enceinte, le capteur au moins est comprimé contre le conteneur et/ou dans laquelle la station d'accueil comporte des moyens (23) pour recevoir des données de mesure transmises sans fil, en particulier du conteneur, et/ou est configurée pour l'alimentation électrique sans fil des capteurs, en particulier dans le conteneur.

4. Station d'accueil selon l'une des revendications précédentes à 3, dans laquelle la station d'accueil possède au moins une connexion pour assurer la connexion et/ ou un réceptacle pour recevoir une sortie de gaz d'échappement d'un conteneur reçu, et dans laquelle cette connexion est reliée de manière fluide, en particulier à un filtre stérile, de telle sorte que le mélange air/vapeur/condensat introduit dans la connexion est conduit à travers le filtre stérile et/ou le réceptacle pour ouvrir et/ ou fermer une soupape de gaz d'échappement du conteneur, en particulier la sortie des gaz d'échappement du conteneur, en particulier pour comprimer et/ou ouvrir un tuyau flexible, en particulier en TPE, et/ou une valve de compression de tuyau du conteneur et/ou contre une valve, la connexion étant reliée de manière fluide à la valve de telle sorte que le mélange air/vapeur/condensat introduit dans la connexion passe sur et/ou à travers la valve.

5. Système comprenant au moins une station d'accueil (20) selon l'une des revendications précédentes 1 à 4 et au moins un conteneur (1), en particulier un conteneur de déchets, comprenant une première cavité (11) fermée sur tous les côtés, dans lequel le conteneur (1) comprend un premier élément chauffant inductif (4).

6. Procédé de décontamination et/ou de stérilisation, dans lequel un conteneur (1), en particulier un conteneur à déchets, comportant une première cavité (11) fermée de tous côtés et un premier élément chauffant inductible (4), est utilisé pour la collecte, en particulier la collecte des déchets, et est ensuite amené dans une station d'accueil selon l'une des revendications 1 à 4 pour la stérilisation et/ou la décontamination, et est éliminé, en particulier incinéré, après la stérilisation et/ou la décontamination.

7. Procédé selon la revendication 6, dans lequel la collecte du contenu du conteneur est effectuée séparément de la station d'accueil.

8. Procédé selon l'une quelconque des revendications précédentes 6 à 7, dans lequel la stérilisation et/ou la décontamination est effectuée en chauffant l'élément chauffant inductible par le champ électromagnétique généré par la station d'accueil, en particulier au moyen de la vapeur générée par l'élément chauffant inductif.

9. Procédé selon l'une quelconque des revendications précédentes 6 à 8, dans lequel la stérilisation et/ou la décontamination constitue une stérilisation et/ou une décontamination de la première cavité et/ou de la matière, en particulier des déchets, reçue dans la première cavité.

10. Procédé selon l'une des revendications précédentes 6 à 9, dans lequel, après la stérilisation et/ou la décontamination, le conteneur est séparé et/ou retiré de la station d'accueil et le conteneur est éliminé et/ou la station d'accueil n'est pas éliminée.

11. Procédé selon l'une quelconque des revendications précédentes 6 à 10, dans lequel la station d'accueil est utilisée, en particulier successivement, pour stériliser et/ou décontaminer plusieurs premières cavités d'un conteneur respectif et/ ou la matière, en particulier les déchets, qu'il contient, les conteneurs comprenant chacun une première cavité (11) fermée de tous les côtés et un premier élément chauffant inductible (4), les conteneurs étant éliminés, en particulier incinérés, après la stérilisation et/ou la décontamination, en particulier après avoir été retirés et/ou séparés de la station d'accueil.

12. Procédé selon l'une des revendications précédentes 6 à 11, dans lequel, au cours de la stérilisation et/ou de la décontamination, au moins une, en particulier plusieurs valeurs de mesure de la pression et/ou de la température est/sont reçues, en particulier demandées, sans fil à partir d'au moins un capteur du conteneur et, en particulier, le champ électromagnétique, en particulier son intensité et/ou sa durée, est contrôlé, en particulier régulé, en particulier par la station d'accueil, au moins également en fonction de ladite au moins une valeur de mesure de la pression et/ou de la température reçues.

13. Procédé selon l'une quelconque des revendications précédentes 6 à 12, dans lequel la collecte est effectuée en alimentant et/ou en aspirant des matières solides et/ou liquides, en particulier des déchets, dans la première cavité.
